# EUROPÄISCHE PATENTSCHRIFT

(11) **EP 0 792 628 B1**
(45) Veröffentlichungstag und Bekanntmachung des Hinweises auf die Patenterteilung: **02.05.2002**
(21) Anmeldenummer: 96118430.6
(22) Anmeldetag: 16.11.1996
(51) Int. Cl.: A61F 2/36

(54) **Endoprothese für ein künstliches Hüftgelenk**
Endoprosthesis for an artificial hip joint
Endoprothèse pour une articulation artificielle de la hanche

(30) Priorität: 29.02.1996 DE 19607609; 24.05.1996 DE 29609287 U
(43) Veröffentlichungstag der Anmeldung: 03.09.1997
(73) Patentinhaber: Scholz, Werner, 30173 Hannover (DE)
(72) Erfinder: Scholz, Werner, 30173 Hannover (DE)
(74) Vertreter: Leine, Sigurd, Dipl.-Ing.

(56) Entgegenhaltungen:
- EP-A- 0 121 491
- EP-A- 0 474 015
- EP-A- 0 610 575
- EP-A- 0 621 019
- CH-A- 660 955
- DE-A- 3 605 630
- DE-A- 3 935 488
- DE-A- 4 445 892
- DE-U- 29 609 287
- FR-A- 2 310 120
- FR-A- 2 349 319
- FR-A- 2 606 628
- FR-A- 2 689 756
- US-A- 3 102 536

## Beschreibung

Die Erfindung betrifft eine Endoprothese der im Oberbegriff des Anspruchs 1 genannten Art für ein künstliches Hüftgelenk.

Durch die Firmendruckschrift "Endoprothesen-System nach Prof. Mittelmeier" der Firma OSTEO AG in CH-2545 Selzach/Schweiz ist eine Endoprothese bekannt, die einen Schaft aufweist, der im wesentlichen über seine gesamte Länge in einen Oberschenkelknochen einsetzbar ist und an seinem freien Ende einen konischen Zapfen aufweist, auf den ein Kugelkopf mit einer komplementär konisch geformten Ausnehmung selbsthemmend aufsetzbar ist. Nach der genannten Firmendruckschrift lassen sich Kugelköpfe unterschiedlicher Gestalt aufsetzen. Zum einen haben die Kugelköpfe unterschiedliche Außendurchmesser, insbesondere sind die konischen Ausnehmungen in bezug zu dem Kugelkopf mit unterschiedlicher axialer Distanz angeordnet, so daß durch Aufsetzen unterschiedlicher Kugelköpfe die Lage des Kugelkopfes in bezug zu dem Zapfen und damit auch zu dem Schaft und dem Oberschenkelknochen anpaßbar ist.

Ein Nachteil dieses bekannten Endoprothesensystems besteht darin, daß mehrere Kugelköpfe mit unterschiedlicher Anordnung der konischen Ausnehmung in bezug zu dem Zentrum des Kugelkopfes vorrätig gehalten werden müssen, um die Lage des Kugelkopfes in bezug zu dem Zapfen und damit zu dem Schaft den jeweiligen Verhältnissen anpassen zu können. Diese Vorratshaltung ist aufwendig und teuer. Hinzu kommt, daß eine Anpassung nur in groben Stufen möglich ist, damit der Umfang der Vorratshaltung nicht zu groß ist.

Durch US 3 102 536 ist eine Endoprothese der im Oberbegriff des Anspruchs 1 genannten Art bekannt, bei der der zylindrische Zapfen in Zapfrichtung gespalten ist und in diesem Bereich eine konische Bohrung mit einem konischen Gewinde aufweist, in die eine konische Madenschraube einschraubbar ist, um so den gespaltenen Bereich des konischen Zapfens zu spreizen und mit seiner Außenfläche gegen die Innenwandung einer zylindrischen Ausnehmung in dem Kugelkopf festzuklemmen. Diese bekannte Endoprothese hat den Nachteil, daß die konische Gewindebohrung in dem zylindrischen Zapfen und das komplementär konische Außengewinde der Madenschraube schwierig herstellbar ist. Außerdem besteht der Nachteil, daß der Kugelkopf in Verlängerung seiner konischen Ausnehmung einen Durchbruch aufweisen muß, um einen Schraubendreher zum Schrauben der Madenschraube hindurchführen zu können. Der Durchbruch stellt eine Unterbrechung in der Oberfläche des Kugelkopfes dar.

Der Erfindung liegt die Aufgabe zugrunde, eine Endoprothese der im Oberbegriff des Anspruchs 1 genannten Art zu schaffen, die einfach und preiswert herzustellen und einfach handzuhaben ist.

Die der Erfindung zugrundeliegende Aufgabe wird durch die im Kennzeichen des Anspruchs 1 angegebene Lehre gelöst.

Der Grundgedanke dieser Lehre besteht darin, die Betätigung der Klemmittel nicht von der dem zylindrischen Zapfen gegenüberliegenden Seite des Kugelkopfes her durchzuführen, was die Durchbrechung der Lageroberfläche des Kugelkopfes bedeutet, sondern von der entgegengesetzten Seite her, also von der Aufsteckseite des Kugelkopfes her. Dies wird erfindungsgemäß ermöglicht durch eine Klemmvorrichtung, die nach Art einer Spannhülse ausgebildet ist, wie sie z.B. in Drehmaschinen zum Festspannen von Rundmaterial üblich ist. Diese Klemmvorrichtung weist bei der erfindungsgemäßen Endoprothese eine Klemmhülse auf, deren Innenfläche zylindrisch und die auf dem zylindrischen Zapfen verschiebbar ist. Ein Ende der Klemmhülse ragt mit einer sich konisch verjüngenden Außenfläche in einen komplementär konisch ausgebildeten Teil der Ausnehmung in dem Kugelkopf, während das andere Ende der Klemmhülse an einer Mutter anliegt, die mit dem Kugelkopf verschraubt ist. Bei der Verwendung eines derartigen Implantats wird die Endoprothese mit aufgesetztem Kugelkopf implantiert und der Kugelkopf durch Verschieben auf dem zylindrischen Zapfen in eine den anatomischen Verhältnissen des Patienten angemessene Lage gebracht, und sobald diese erreicht ist, wird die Mutter angezogen und somit die Klemmhülse in den konischen Spalt zwischen dem zylindrischen Zapfen und der konischen Innenfläche der Ausnehmung in dem Kugelkopf gepreßt und somit durch Keilwirkung ein völlig sicheres Festspannen erzielt.

Wenn der Sitz der Klemmhülse auf dem zylindrischen Zapfen so gestaltet ist, daß eine leichte Verschiebbarkeit gegeben ist, so muß beim Festziehen der konische Teil der Klemmhülse zusammengepreßt werden. Dazu ist eine größere Kraft erforderlich, die zusätzlich beim Anziehen der Mutter aufgebracht werden muß. Zur Vermeidung dieser zusätzlichen Kraftaufwendung ist gemäß einer Weiterbildung der Erfindung die Klemmhülse wenigstens im Bereich der sich konisch verjüngenden Außenfläche axial geschlitzt. Dadurch kann sich die Klemmhülse beim Festziehen ohne Kraftaufwand fest um den zylindrischen Zapfen legen, wenn die Mutter festgezogen und so das konische Ende der Klemmhülse keilförmig in den komplementär konisch ausgebildeten Teil der Ausnehmung in dem Kugelkopf eingedrückt wird.

Die Mutter ist zweckmäßigerweise als Überwurfmutter ausgebildet, die auf einen Fortsatz des Kugelkopfes aufgeschraubt ist. Besonders zweckmäßig ist es dabei, daß die Überwurfmutter in beiden Axialrichtungen formschlüssig mit der Klemmhülse verbunden ist. Das bedeutet, daß die Klemmhülse bei jeder Schraubbewegung der Axialbewegung der Überwurfmutter folgt, also beim Festziehen nicht nur eingedrückt, sondern beim Lösen der überwurfmutter auch wieder aus der Keilverbindung herausgezogen wird. Diese Eigenschaft ist besonders vorteilhaft beim "Anprobieren", wenn also die günstigste anatomische Lage gefunden werden soll. In jeder beliebigen Einstellage ist leicht ein Festspannen und Wiederlösen möglich.

Zum Festziehen der Mutter müssen natürlich Schraubwerkzeuge ansetzbar sein, wobei gleichzeitig auch zweckmäßigerweise an dem Kugelkopf oder einem Fortsatz daran Schraubwerkzeuge ansetzbar sein sollten. Zu diesem Zwecke können z. B. Abflachungen vorgesehen sein. Besonders zweckmäßig ist es jedoch, wenn in der Außenfläche der Überwurfmutter und/oder der Außenfläche des Fortsatzes des Kugelkopfes eine Bohrung, vorzugsweise eine Sackbohrung zum Ansetzen eines Hakenschlüssels vorgesehen ist.

Um die erfindungsgemäße Lehre auch bei den eingangs zum Stand der Technik beschriebenen Endoprothesen für ein künstliches Hüftgelenk anwenden zu können, bei denen sich am freien Ende des Schaftes ein konischer Zapfen befindet, auf den noch ein Kugelkopf mit einer komplementär konisch geformten Ausnehmung selbsthemmend aufsetzbar ist, sieht eine Weiterbildung der Erfindung vor, daß der Zapfen aus einem gesonderten Teil besteht und an seinem dem Schaft zugewandten Ende eine Ausnehmung aufweist, die komplementär konisch zu einem am Ende des Schaftes angeordneten Konuszapfen ausgebildet ist, derart, daß der Zapfen selbsthemmend auf dem Konuszapfen aufsteckbar ist. Auf diese Weise ist es möglich, auf den Konuszapfen eines bekannten Schaftes einen als gesondertes Teil ausgebildeten Zapfen aufzusetzen, so daß dieser Zapfen dann mit seiner zylindrischen Außenfläche die axiale Verstellung des Kugelkopfes ermöglicht.

Anhand der Zeichnung soll die Erfindung an Ausführungsbeispielen näher erläutert werden.
- Fig. 1: zeigt ein Ausführungsbeispiel, das nicht der Erfindung entspricht.
- Fig. 2: zeigt ein Ausführungsbeispiel der Erfindung in vergrößerter Darstellung und teilweise geschnitten und weggeschnitten, und
- Fig. 3: zeigt eine Abwandlung der Ausführungsform gemäß Fig. 2.

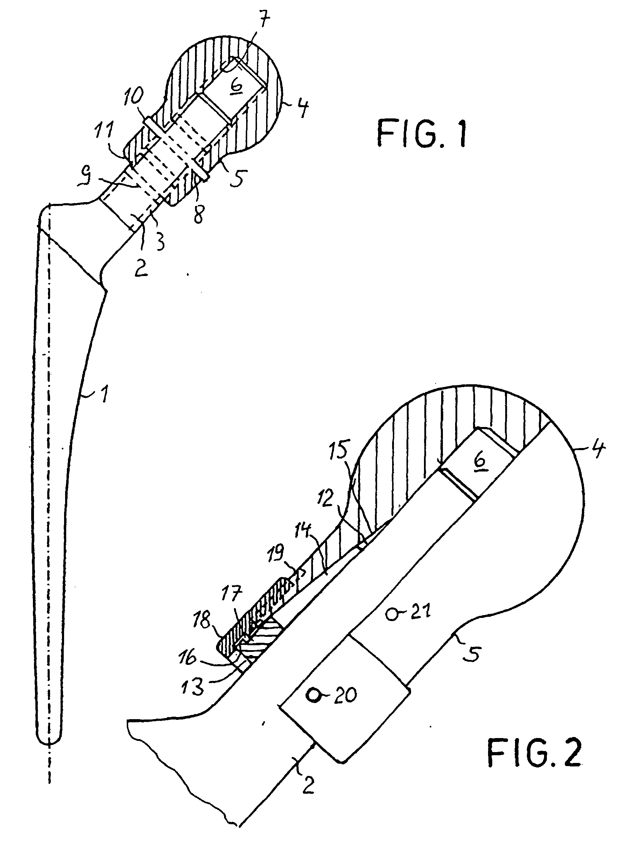

Fig. 1 zeigt eine Endoprothese mit einem in einen Oberschenkelknochen einzusetzenden Schaft 1, an dem sich ein zylindrischer Zapfen 2 befindet, der ein Außengewinde 3 aufweist. In einem Kugelkopf 4 und dessen Fortsatz 5 befindet sich eine zylindrische Ausnehmung 6 mit einem Innengewinde 7.

In dem Fortsatz 5 befindet sich eine Querbohrung 8, während sich in dem zylindrischen Zapfen 2 Querbohrungen 9 befinden. In der dargestellten Lage fluchtet die Querbohrung 8 in dem Fortsatz 5 mit einer der Querbohrungen 9 in dem zylindrischen Zapfen 2, und in dieser Lage ist ein Sicherungsstift 10 in die beiden genannten Bohrungen 8 und 9 eingepreßt. Dadurch ist der Kugelkopf 4 in der dargestellten Schraublage gegen Verdrehungen und damit auch gegen axiale Veränderungen in bezug zu dem Zapfen 2 gesichert.

Aus Fig. 1 ist ersichtlich, daß die vordere Stirnkante 11 des Fortsatzes 5 von der Querbohrung 8 einen Abstand hat, der dem Zweifachen der untereinander gleichen Abstände zwischen den Querbohrungen 9 entspricht. Diese Bemessung führt dazu, daß immer dann, wenn die vordere Stirnkante 11 des Fortsatzes 5 mit einer Querbohrung 9 in dem Zapfen 2 fluchtet, auch die Querbohrung 8 in dem Fortsatz 5 mit einer der Querbohrungen 9 fluchtet und somit in dieser Lage ein Sicherungsstift 10 einpreßbar ist. Dadurch erübrigt sich unnötiges Suchen der jeweiligen fluchtenden Stellungen.

Bei Gebrauch wird zunächst die Endoprothese mit ihrem Schaft 1 in den entsprechend vorbereiteten Oberschenkelknochen eingesetzt, wobei der Kugelkopf 4 bereits in einer beliebigen Schraublage aufgeschraubt sein kann. Dann wird durch Verdrehen des Kugelkopfes 4 dessen gewünschte Lage in bezug zu dem Zapfen 2 und damit auch zu dem Schaft 1 eingestellt, wobei allerdings diese Einstellung nur in Stufen möglich ist, in denen jeweils die Querbohrung 8 mit einer der Querbohrungen 9 fluchtet, was durch Fluchten der vorderen Stirnkante 11 mit einer der Querbohrungen 9 sichtbar ist.

Fig. 2 zeigt ein Ausführungsbeispiel der Erfindung und entspricht im wesentlichen dem oberen Teil der Darstellung in Fig. 1, wobei gleiche Teile mit gleichen Bezugszeichen versehen sind. Die Darstellung in Fig. 2 ist vergrößert, und der Kugelkopf 4 mit seinem Fortsatz 5 und den übrigen noch zu erläuternden Teilen ist zur Hälfte geschnitten dargestellt.

Aus Fig. 2 ist ersichtlich, daß die Oberfläche des Zapfens 2 glatt ist, ebenso wie die Innenfläche der zylindrischen Ausnehmung 6. Das bedeutet, daß der Kugelkopf 4 grundsätzlich auf dem Zapfen 2 axial verschieblich ist.

Die zylindrische Ausnehmung geht im Bereich des Fortsatzes 5 in eine konische Ausnehmung 12 über, in die eine Klemmhülse 13 mit einem geschlitzten konischen Ende 14 ragt, dessen Außenfläche 15 komplementär zu der Innenfläche der konischen Ausnehmung 12 ausgestaltet ist.

Die Klemmhülse 13 weist einen äußeren, radialen Kragen 16 auf, der formschlüssig in eine Innennut 17 einer überwurfmutter 18 eingreift, die auf ein Außengewinde 19 des Fortsatzes 5 aufgeschraubt ist. Die Klemmhülse 13 ist somit in bezug auf axiale Bewegungen formschlüssig und in bezug auf Drehbewegungen beweglich mit der Überwurfmutter 18 verbunden.

Durch Festziehen der Überwurfmutter 18 wird die Klemmhülse 13 mit ihrem geschlitzten konischen Ende 14 in die konische Ausnehmung 12 eingepreßt, so daß sie den zylindrischen Zapfen 2 gegenüber dem Fortsatz 5 verkeilt. Dadurch ist der Kugelkopf 4 in der jeweiligen Axiallage fest mit dem Zapfen 2 verbunden.

Diese Verbindung kann in einfacher Weise gelöst werden, indem die Überwurfmutter 18 wieder gelöst wird, wobei sie die Klemmhülse 13 mittels ihres Kragens 16 wieder aus der Keillage herauszieht.

Zum Festziehen und Lösen der überwurfmutter 18 ist in dieser eine Sackbohrung 20 zum Ansetzen eines Hakenschlüssels vorgesehen. Zur Aufnahme der Reaktionskraft kann ein entsprechender Hakenschlüssel in eine Sackbohrung 21 in dem Fortsatz 5 eingesetzt werden.

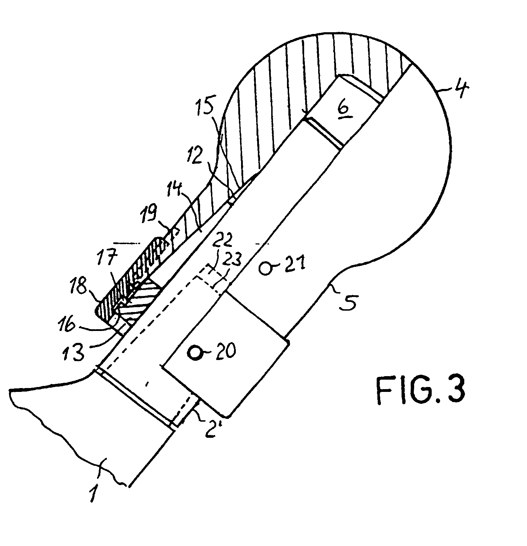

Fig. 3 zeigt eine Abwandlung der Ausführungsform gemäß Fig. 2. Gleiche Teile sind mit gleichen Bezugsziffern versehen. Die Abwandlung besteht darin, daß der Zapfen 2' ein gesondertes Teil bildet, daß an seinem dem Schaft 1 zugewandten Ende eine Ausnehmung 22 aufweist, die komplementär konisch zu einem am Ende des Schaftes 1 angeordneten Konuszapfen 23 ausgebildet ist. Der Zapfen 2' ist somit selbsthemmend auf den Konuszapfen 23 aufsteckbar, wozu der Konuswinkel natürlich in jedem Fachmann bekannter Weise entsprechend gewählt sein muß.

Bei dieser Ausführungsform gemäß Fig. 3 kann es sich bei dem Schaft 1 mit dem Konuszapfen 23 um die allgemein gebräuchliche bekannte Ausführungsform handeln. Durch Aufstekken des Zapfens 2' auf diesen bekannten Schaft ergibt sich dann eine Ausführungsform, bei der das Prinzip der Erfindung anwendbar ist, den Kugelkopf 4 in Axialrichtung des Zapfens 2' feststellen zu können.

## Patentansprüche

1. Endoprothese für ein künstliches Hüftgelenk,
mit einem Schaft (1),
mit einem zylindrischen Zapfen (2) an einem Ende des Schaftes (1),
mit einem Kugelkopf (4), in dem sich eine Ausnehmung befindet, in die der zylindrische Zapfen (2) hineinragt und
mit einer Klemmvorrichtung zur Halterung des Kugelkopfes (4) in verschiedenen Axiallagen in bezug zu dem Zapfen (2),
**dadurch gekennzeichnet, daß** die Klemmvorrichtung nach Art einer Spannhülse ausgebildet ist, die eine Klemmhülse (13) aufweist, deren Innenfläche zylindrisch und die auf dem Zapfen (2) verschieblich ist, daß ein Ende (14) der Klemmhülse (13) mit einer sich konisch verjüngenden Außenfläche (15) in einen komplementär konisch ausgebildeten Teil der Ausnehmung in den Kugelkopf (4) hineinragt, während das andere Ende der Klemmhülse (13) an einer Mutter anliegt, die mit dem Kugelkopf (4) verschraubt ist.

2. Endoprothese nach Anspruch 1, **dadurch gekennzeichnet, daß** die Klemmhülse (13) wenigstens im Bereich der sich konisch verjüngenden Außenfläche (15) axial geschlitzt ist.

3. Endoprothese nach Anspruch 1, **dadurch gekennzeichnet, daß** die Mutter eine Überwurfmutter (18) ist, die auf einen Fortsatz (5) des Kugelkopfes (4) aufgeschraubt ist.

4. Endoprothese nach Anspruch 1, **dadurch gekennzeichnet, daß** die Überwurfmutter (18) in beiden Axialrichtungen formschlüssig mit der Klemmhülse verbunden ist.

5. Endoprothese nach Anspruch 1, **dadurch gekennzeichnet, daß** sich in der Außenfläche der Überwurfmutter (18) wenigstens eine Bohrung, vorzugsweise eine Sackbohrung (20) zum Ansetzen eines Hakenschlüssels befindet.

6. Endoprothese nach Anspruch 3, **dadurch gekennzeichnet, daß** sich in der Außenfläche des Fortsatzes (5) des Kugelkopfes (4) eine Bohrung, vorzugsweise eine Sackbohrung (21) zum Ansetzen eines Hakenschlüssels befindet.

7. Endoprothese nach Anspruch 1, **dadurch gekennzeichnet, daß** der Zapfen (2') aus einem gesonderten Teil besteht und am seinem dem Schaft (1) zugewandten Ende eine Ausnehmung (22) aufweist, die komplementär konisch zu einem am Ende des Schaftes (1) angeordneten Konuszapfen (23) ausgebildet ist, derart, daß der Zapfen (2') selbsthemmend auf den Konunszapfen (23) aufsteckbar ist.

## Claims

1. Endoprosthesis for an artificial hip joint, having a shank (1) with a cylindrical pin (2) at one end of the shank (1), having a spherical head (4) in which a recess is found, into which the cylindrical pin (2) projects and
having a clamping device for retaining the spherical head (4) in various axial positions in relation to the pin (2),
**characterised in that** the clamping device is constructed in the form of a split taper sleeve, having a clamping sleeve (13) whose inner surface is cylindrical and which is displaceable on the pin (2), that one end (14) of clamping sleeve (13) having a conically tapering outer surface (15), projects into a complimentarily conically constructed part of the recess in spherical head (4), whilst the other end of clamping sleeve (13) abuts with a nut which is screwed together with spherical head (4).

2. Endoprosthesis according to claim 1, **characterised in that** clamping sleeve (13) is axially slotted at least in the region of the conically tapering outer surface (15).

3. Endoprosthesis according to claim 1, **characterised in that** the nut is a union nut (18), which is screwed onto an extension (5) of spherical head (4).

4. Endoprosthesis according to claim 1, **characterised in that** union nut (18) is form-lockingly connected to the clamping sleeve in both axial directions.

5. Endoprosthesis according to claim 1, **characterised in that** at least one bore, preferably a pocket bore (20) is found in the outer surface of union nut (18) for attaching a hooked wrench.

6. Endoprosthesis according to claim 1, **characterised in that** at least one bore, preferably a pocket bore (21) is found in the outer surface of the extension (5) of spherical head (4) for attaching a hooked wrench.

7. Endoprosthesis according to claim 1, **characterised in that** pin (2') is comprised of an isolated part and has a recess (22) on its end turned towards shank (1), which is conically constructed complimentary to a conical pin (23) arranged at the end of shank (1), in such a way that pin (2') is self-lockingly attachable to conical pin (23).

## Revendications

1. Endoprothèse pour une articulation artificielle de la hanche,
avec une tige (1),
avec une broche cylindrique (2) à une extrémité de la tige (1),
avec une tête sphérique (4) dans laquelle se trouve un évidement dans lequel la broche cylindrique (2) est engagée et
avec un dispositif de blocage pour maintenir la tête sphérique (4) dans différentes positions axiales par rapport à la broche (2),
**caractérisée en ce que** le dispositif de blocage est réalisé à la manière d'un manchon de serrage qui présente une douille de blocage (13) dont la surface intérieure est cylindrique et qui est déplaçable sur la broche (2), **en ce qu'**une extrémité (14) de la douille de blocage (13) est engagée avec sa surface extérieure se rétrécissant de manière conique dans une partie complémentaire réalisée de manière conique de l'évidement dans la tête sphérique (4) tandis que l'autre extrémité de la douille de blocage (13) est en appui sur un écrou qui est vissé sur la tête sphérique (4).

2. Endoprothèse selon la revendication 1,
**caractérisée en ce que** la douille de blocage (13) est fendue axialement au moins dans la zone de la surface extérieure (15) se rétrécissant de manière conique.

3. Endoprothèse selon la revendication 1,
**caractérisée en ce que** l'écrou est un écrou d'accouplement (18) qui est vissé sur un prolongement (5) de la tête sphérique (4).

4. Endoprothèse selon la revendication 1,
**caractérisée en ce que** l'écrou d'accouplement (18) est relié à la douille de blocage par concordance de forme selon les deux directions axiales.

5. Endoprothèse selon la revendication 1,
**caractérisée en ce qu'**au moins un trou, de préférence un trou borgne (20), est pratiqué dans la surface extérieure de l'écrou d'accouplement (18) pour la mise en place d'une clé à ergot.

6. Endoprothèse selon la revendication 3,
**caractérisée en ce qu'**un trou, de préférence un trou borgne (21), est pratiqué dans la surface extérieure du prolongement (5) de la tête sphérique (4) pour la mise en place d'une clé à ergot.

7. Endoprothèse selon la revendication 1,
**caractérisée en ce que** la broche (2') est constituée par une pièce rapportée et présente à son extrémité tournée vers la tige (1) un évidement (22) qui est réalisé conique de manière complémentaire à une broche en cône (23) disposée à l'extrémité de la tige (1) de telle sorte que la broche (2') est emboîtable de manière autobloquante sur la broche en cône (23).
